(19) Europäisches Patentamt
European Patent Office
Office européen des brevets

(11) Veröffentlichungsnummer: **0 001 277**
**B1**

(12) **EUROPÄISCHE PATENTSCHRIFT**

(21) Anmeldenummer: **78100918.8**

(22) Anmeldetag: **18.09.78**

(51) Int. Cl.³: **C 07 C 143/70,**
C 07 C 139/02

# (54) Verfahren zur Herstellung von Sulfonsäurechloriden

(30) Priorität: **28.09.77 DE 2743541**

(43) Veröffentlichungstag der Anmeldung:
**04.04.79 Patentblatt 79/07**

(45) Bekanntmachung des Hinweises auf die Patenterteilung:
**12.11.80 Patentblatt 80/23**

(84) Benannte Vertragsstaaten:
**BE CH DE FR GB LU NL**

(56) Entgegenhaltungen:
**DE - A - 1 468 136**
**DE - A - 2 635 279**
**DE - A - 2 635 281**
**US - A - 3 772 373**
**Houben-Weyl "Methoden der organischen Chemie" 4.Auflage, Band IX, 1955, Georg Thieme Verlag, Stuttgart, Seiten 567—569**
**Ullmanns Encyklopädie der technischen Chemie, 4.Auflage, Band 8, 1974, Verlag Chemie, Weinheim, Seite 420**

(73) Patentinhaber: **BAYER AG**
**ZENTRALBEREICH PATENTE, MARKEN UND LIZENZEN**
**D-5090 LEVERKUSEN 1, BAYERWERK (DE)**

(72) Erfinder: **Pfister, Theodor, Dr.**
**Siller-Strasse 51**
**D-5600 Wuppertal-1 (DE)**
**Schenk, Wolfgang, Dr.**
**Odenthaler Strasse 62/64**
**D-5090 Leverkusen 1 (DE)**
**Blank, Heinz Ulrich, Dr.**
**Am Geusfelde 35**
**D-5068 Odenthal (DE)**

**EP 0 001 277 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von sulfonsäurechloriden

Die Erfindung betrifft ein Verfahren zur Herstellung von Sulfonsäurechloriden durch Umsetzung von aromatischen Verbindungen mit Chlorsulfonsäure und Phosgen.

Es ist bekannt (Ullmanns Enzyklopädie der technischen Chemie, 4. Auflage, Band 8 (1974), Seite 420) Benzolsulfonsäurechlorid durch Umsetzung von Benzol mit überschüssiger Chlorsulfonsäure herzustellen. Die Verwendung überschüssiger Chlorsulfonsäure ist desonders im Hindblick auf den Umweltschutz ein erheblicher Nachteil dieses Verfahrens, da die Überschüssige Chlorsulfonsäure bei der Aufarbeitung des Reaktionsgemisches mit Wasser zu Chlorwasserstoff und Schwefelsäure hydrolysiert wird und zusammen mit der zusätzlich auch als Nebenprodukt entstehenden Schwefelsäure, sowie zusammen mit erheblichen Mengen nicht zum Sulfonsäurechlorid umgesetzter Benzolsulfonsäure als sogenannte Dünnsäure erhalten wird. Die Aufarbeitung und Beseitigung dieses Zwangsanfalls an Dünnsäure führt zu erheblichen Aufwendungen. Bei einfacher Neutralisierung der Dünnsäure ergibt sich ein entsprechender Salzgehalt des Abwassers, der ebenfalls uas Gründen des Umweltschutzes unerwünscht ist und eine aufwendige Entsalzung des Abwassers notwendig macht.

Es wurde ein Verfahren zur Herstellung von Sulfonsäurechloriden der Formel

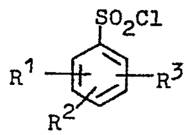

(I)

worin

$R^1$, $R^2$ und $R^3$ gleich oder verschieden sind und Wasserstoff, einen niederen Alkyl- oder einen Cycloalkylrest, Halogen, Aryl, Aralkyl, Aryläther oder einen Rest $—SO_2Cl$, $—SO_2$-Aryl,

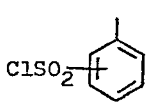 , 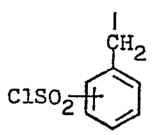 , 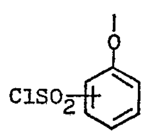 oder 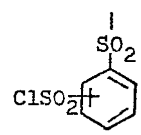

bedeuten

oder wo benachbarte Reste

$R^1$ und $R^2$ zu einem gegebenenfalls durch eine Sulfonsäurechloridgruppe substituierten cycloaliphatischen, aromatischen oder heteroaromatischen Ring verknüpft sind, durch Umsetzung von aromatischen Verbindungen der Formel

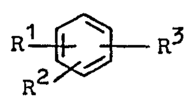

(II)

worin

$R^1$, $R^2$ und $R^3$ die oben genannte Bedeutung haben, mit Chlorsulfonsäure und Phosgen gefunden, bei dem man zuerst die aromatische Verbindung mit Chlorosulfonsäure in einer molaren Menge von etwa 1:1 bis zu 1:1,2, gegebenenfalls in Gegenwart eines Lösungsmittels und gegebenenfalls in Gegenwart einer Verbindung, die gegenüber Chlorsulfonsäure als Lewis-Base wirksam werden kann, zur Reaktion bringt und dann in Gegenwart eines an sich bekannten Katalysators mit Phosgen umsetzt.

Niedere Alkylreste ($R^1$ bis $R^3$) können geradkettige oder verzweigte Alkylreste mit 1 bis 6, bevorzugt 1 bis 4, Kohlenstoffatomen sein. Beispielsweise seien genannt: Methyl, Ethyl, Propyl, Isopropyl, Butyl, Isobutyl, Pentyl, Hexyl und Isohexyl.

Als Cycloalkylreste ($R^1$ bis $R^3$ seien beispielsweise Cyclopentyl und Cyclohexyl, bevorzugt Cyclohexyl, genannt.

Als Halogene ($R^1$ bis $R^3$) seien Fluor, Chlor, Brom und Jod bevorzugt Fluor, Chlor und Brom, genannt.

Als Arylrest ($R^1$ bis $R^3$) sei beispielsweise Phenyl genannt.

Als Aralkylreste ($R^1$ bis $R^3$) kommen beispielsweise solche mit 6 bis 18 Kohlenstoffatomen, deren aliphatischer Teil 1 bis 6 Kohlenstoffatome enthält und deren aromatischer Teil einen Rest aus der Benzolreihe darstellt, infrage. Beispielsweise seien die folgenden araliphatischen Reste genannt:

Benzyl, $\beta$-Ethyl-phenyl, $\gamma$-Phenyl-propyl und $\beta$-Phenyl-n-hexyl, bevorzugt Benzyl.

Als Aryletherrest ($R^1$ bis $R^3$) sei insbesondere der Phenoxyrest genannt.

Durch Verknüpfung der benachbarten Reste $R^1$ und $R^2$ zu einem cycloaliphatischen oder aromatischen Ring entstehen kondensiert Ringsysteme, wie Indan, Tetralin und Napthalin bevorzugt Naphthalin.

Es ist selbstverständlich möglich, daß die Reste $R^1$ bis $R^3$ durch weitere Reste substituiert sind. Bespielsweise sei halogen, Niederalkyl, Aryl, Aroxy, Alkoxy und Aralkyl genannt.

Als bevorzugte aromatische Verbindungen seien Verbindungen der Formel

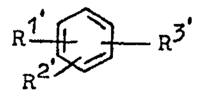

(III)

worin

$R^{1'}$, $R^{2'}$ und $R^{3'}$ gleich oder verschieden sind und Wasserstoff, einen Alkylrest mit 1 bis 4 Kohlenstoffatomen, Fluor, Chlor, Brom, Phenyl, Phenoxy, —$SO_2$-Phenyl, —$SO_2Cl$ oder Benzyl bedeuten oder wo benachbarte Reste

$R^{1'}$ und $R^{2'}$ zu einem cycloaliphatischen, aromatischen oder heteroaromatischen Ring mit 5 oder 6 Ringgliedern verknüpft sind,

genannt.

Beispielsweise seien die folgenden aromatischen Verbindungen genannt:

Benzol, Toluol, Äthylbenzol, Isopropylbenzol (Cumol), Tetralin, o-Xylol, m-Xylol, p-Xylol, Diphenyl, Diphenylmethan, Chlorbenzol, 1-Chlornaphthalin, 2-Chlornaphthalin, o-Chlortoluol, 1,2- 1,3- und 1,4-Dichlorbenzol, 2,3-, 2,4-, 2,5-, 3,4- und 2,6-Dichlor-toluol, 2,3-, 2,4-, 2,6-, 3,4- und 2,5-Dimethylchlorbenzol, Brombenzol, Fluorbenzol, 1,2,3- und 1,2,4-Trichlorbenzol, Diphenyläther, Naphthalin, 1- und 2-Methylnaphthalin und 2-, und 3- und 4-Bromtoluol, Diphenylsulfon, Benzolsulfochlorid, Toluolsulfochlorid, Diphenylensulfon, Diphenylenoxyd.

Insbesondere wird Benzol als Ausgangsverbindung bevorzugt. Das erfindungsgemäße Verfahren wird Vorzugsweise in Gegenwart von Sulfonierungschilfsmitteln durchgeführt. Sulfonierungshilfsmittel nach dem erfindungsgemäßen Verfahren sind im allgemeinen Verbindungen, die gegenüber Chlorsulfonsäure als Lewis-Basen wirksam werden können.

Als Sulfonierungshilfsmittel für das erfindungsgemäße Verfahren seinen beispielsweise genannt:

1. Carbonsäuren und deren Derivate wie z.B. Essigsäure, Propionsäure, Buttersäure, Pivalinsäure, Valeriansäure, Capronsäure und Adipinsäure, halogenierte Carbonsäuren wie Mono-, Di- und Trichloressigsäure, Carbonsäureanhydride wie z.B. Acetanhydrid, Propionsäureanhydrid, Bernsteinsäureanhydrid und Phthalsäureanhydrid, Carbonsäurechloride wie z.B. Acetylchlorid, Propionsäurechlorid, Buttersäurechlorid und Adipinsäuredichlorid, Carbonsäureester wie z.B. Essigsäuremethyl- und äthylester, Carbonsäureamide wie z.B. Formamid, Dimethylformamid, Phthalimid, succinimid,

2. Harnstoff und Alkylharnstoffe,

3. Phosphorsäure und organische Phosphorverbindungen wie z.B. Trimethyl-, Triäthyl- und Tributyl phosphat, Trimethyl- und Triäthylphosphit,

4. Äther wie z.B. Diäthyläther, Tetrahydrofuran und Dioxan,

5. Ketone wie z.B. Aceton, Methyläthylketon, Methylpropylketon, Methylisobutylketon, cyclopentanon und Cyclohexanon und

6. Amine wie z.B. Mono-, Di- und Triethylamin, Isopropylamin, tert.-Butylamin, Cyclohexylamin, Ethanolamin, Morpholin, Pyridin und Imidazol.

Besonders bevorzugt werden Carbonsäuren bzw. deren Derivate und Phosphorverbindungen, insbesondere bevorzugt werden Dimethylformamid, Essigsäure und Phosphorsäure.

Das Sulfonierungshilfsmittel kann in einer Menge von 0,5 bis 10 Mol-%, vorzugsweise von 1 bis 8 Mol-% und insbesondere von 2 bis 5 Mol-% angewandt werden; im allgemeinen kann es zu Beginn der Reaktion ganz oder teilweise mit vorgelegt werden. Der gegebenenfalls nicht eingesetzte Teil wird dann im weiteren Verlauf der ersten Reaktionsstufe mit zugetropft bzw. portionweise zugegeben.

Nach dem erfindungsgemäßen Verfahren wird beispielsweise in der ersten Verfahrensstufe die Chlorsulfonsäure zusammen mit dem Sulfonierungshilfsmittel vorgelegt und die aromatische Verbindung nach Maßgabe der Reaktionsgeschwindigkeit, die anhand der Chlorwasserstoffentwicklung festgestellt werden cann, zugegeben.

Die Chlorsulfonsäure wird im allgemeinen in etwa molaren Mengen, bezogen auf die Zahl der einzuführenden Sulfonsäurechloridgruppen eingesetzt. Es kann jedoch vorteilhaft sein, einen geringen Überschuß bis zu 20 Mol-% und insbesondere bis zu 5 Mol-%, einzusetzen.

Der erste Verfahrensschritt des erfindungsgemäßen Verfahrens wird im allgemeinen im Temperaturbereich von etwa 0 bis 150°C vorzugsweise von 10 bis 140°C, insbesondere bevorzugt von 20 bis 100°C, in Gegenwart eines Sulfonierungshilfsmittels durchgeführt.

Die Reaktionsgemische aus den aromatischen Verbindungen und Chlorsulfonsäure sind im angegebenen Temperaturbereich auch nach der Umsetzung meistens flüssig und leicht rührbar. Daher ist

die Verwendung eines gegenüber Chlorsulfonsäure inerten Lösungsmittels zwar möglich, aber gewöhnlich nicht erforderlich.

Als Lösungs- oder Verdünnungsmittel für das erfindungsgemäße Verfahren kommen flüssiges Schwefeldioxid, Kohlenwasserstoffe und Halogenkohlenwasserstoffe, insbesonder Halogenalkane, wie z.B. Di- Tri- und Tetrachlormethan, Di-, Tri-und Tetrachloräthylen, Di- Tri-, Tetra- und Pentachloräthan, 1,1,2-Trichlor-1,2,2-trifluoräthan und Tetrafluoräthylen in Betracht.

Die erste Verfahrensstufe des erfindungsgemäßen Verfahrens wird bevorzugt bei Normaldruck ausgeführt; sie kann aber auch bei vermindertem Druck oder unter erhöhtem Druck bis zu 10 Bar, insbesondere bis zu 5 Bar, durchgeführt werden. Ist die Reaktionstemperatur höher als bei Rückflußbedingungen bei Normaldruck, so arbeitet man zweckmäßig bei einem Druck, der dem Dampfdruck des Reaktionsgemisches bei den jeweils gewählten Bedingungen entspricht.

Bei Vorlegen der Chlorsulfonsäure kann bis etwa 100°C auch drucklos durch Eintropfen der aromatischen Verbindung, bzw. durch Einleiten von der gasförmigen aromatischen Verbindung gearbeitet werden.

Nicht umgesetzte aromatische Verbindung kann praktisch quantitativ durch Destillation zurückgewonnen werden.

Die zweite Verfahrensstufe kann direkt im Anschluß an die erste Verfahrensstufe, gewöhnlich ebenfalls ohne Verwendung von Lösungsmitteln durchgeführt werden. Da eine Isolierung der Zwischenprodukte nich erforderlich ist, kann das erfindungsgemäße Verfahren als "Eintopfreaktion" durchgeführt werden.

In der zweiten Verfahrensstufe wird im allgemeinen das Phosgen im Überschuß über die stöchiometrisch erforderliche Menge von 1 Mol Phosgen je Mol der aromatischen Verbindung verwendet. Gewöhnlich wird ein Überschuß von bis zu 100 Mol-%, bevorzugt von 10 bis 50 Mol-%, Phosgen eingesetzt. Selbstverständlich können auch größere Phosgenüberschüsse verwendet werden, jedoch bringt dies im allgemeinen keinen Vorteil. Nicht umgesetztes, überschüssiges Phosgen kann zurückgewonnen und wiederverwendet werden.

Die zweite Verfahrensstufe des erfindungsgemäßen Verfahrens wird in Gegenwart eines Katalysators durchgeführt. Phosgenierungskatalysatoren sind an sich bekannt (US—PS 3673247, FR—PS 1513766, DT—OS 1 593 906, DT—OS 19 63 383 und DT—OS 22 40 883). Beispielsweise seien die folgenden Phosgenierungskatalysatoren genannt: Dimethylformamid, Dimethylacetamid, N-Methylpyrrolidon, Triäthylamin, N,N-Dimethylanilin, Pyridin, Triphenylphosphin und Triphenylphosphinoxid.

Vorzugsweise verwendet man Dimethylformamid.

Die zweite Verfahrensstufe des erfindungsgemäßen Verfahrens, d.h. die Umsetzung von Phosgen mit dem Reaktionsgemisch aus der ersten Verfahrensstufe, wird so durch- geführt, daß das Phosgen während der Reaktion stets im Uberschuß gegenüber der in der ersten Verfahrensstufe gebildeten Sulfonsäure in der Reaktionsmischung vorhanden ist.

Die Umsetzung mit Phosgen wird im allgemeinen im Temperaturbereich von 0 bis 100°C, vorzugsweise von 20 bis 80°C, insbesondere von 40 bis 70°C, durchgeführt. Die zweite Verfahrensstufe wird ebenfalls bevorzugt bei Normaldruck ausgeführt sie kann aber auch bei vermindertem Druck oder unter erhöhtem Druck durchgeführt werden. Bei Verwendung von niedrig siedenden Lösungsmitteln oder von einem großen Überschuß von Phosgen arbeitet man bei Rückfluß unter einem der erforderlichen Sumpftemperatur engepaßten Druck.

Phosgen, das im Abgas der zweiten Reaktionsstufe enthalten ist, kann beispielsweise durch Waschen mit Dichlorbenzol zurückgewonnen werden. Es kann aber auch von Vorteil sein, die Abgaswäsche zusätzlich oder ausschließlich mit Sulfonsäure und/oder Sulfochlorid vorzunehmen und das Phosgen beispielsweise zusammen mit Sulfonsäure zurückzuführen.

Nach Beendigung der Reaktion mit Phosgen, erkennbar am Aufhören der Gasentwicklung oder mit bekannten analytischen Methoden werden leichter flüchtige Komponenten im schwachen Vakuum oder durch Einleiten eines inerten Gases verdrängt.

Nicht umgesetzte aromatische Verbindung und gegebenenfalls das Lösungsmittel wird vorzugsweise durch Destillation abgetrennt und ebenso wie das überschüssige Phosgen wiederverwendet.

Das verbleibende Sulfonsäurechlorid wird entweder als Rohprodukt verwendet oder, falls erforderlich, durch Vakuumdestillation oder Kristallisation gereinigt. Das erfindungsgemäße Verfahren kann sowohl diskontinuierlich als auch kontinuierlich durchgeführt werden.

In einer bevorzugten Herstellung, beispielsweise des Benzolsulfonsäurechlorides oder des Diphenylätherdisulfochlorids, kann man bezüglich der Stufe 1

a) den Aromaten gegebenenfalls zusammen mit den Sulfonierungshilfsstoffen, letztere vollständig oder teilweise, vorlegen und die Chlorsulfonsäure eventuell mit der Restmenge des sulfonierungshilfsstoffs nach Maßgabe der Reaktionsgeschwindigkeit zugegeben oder

b) in einer bevorzugten Arbeitsweise die Chlorsulfonsäure und gegebenenfalls die Sulfonierungshilfsstoffe jeweils ganz oder teilweise vorlegen und den Aromaten, gegebenenfalls synchron mit der restilichen Chlorsulfonsäure und den Sulfonierungshilfsstoffen, nach Maßgabe der Reaktionsgeschwindigkeit zugeben.

Bei der Herstellung von Benzolsulfonsäurechlorid wird die Stufe 1 vorzugsweise bei 20—80°C, insbesondere bei 40—70°C durchgeführt.

Überraschenderweise findet man für die Gesamtreaktion (Stufe 1 und Stufe 2) eine Abnahme der Sulfonbildung unter gleichzeitiger Ausbeutesteigerung an Benzolsulfonsäurechlorid, wenn in Stufe 1 in Gegenwart von Sulfonierungshilfsstoffen bei erhöhter Temperatur umgesetzt wird. In Abwesenheit der Sulfonierungshilfstoffe sinkt dagegen bei erhöhter Temperatur die Ausbeute an Benzolsulfonsäurechlorid ab.

Der als Nebenprodukt anfallende Chlorwasserstoff wird abgeleitet und kann gegebenenfalls isoliert werden. Nach Ablauf der ersten Reaktionsstufe, erkennbar am Ende der Gasentwicklung, kann die nicht umgesetzte aromatische Verbindung und gegebenenfalls das Lösungsmittel abdestilliert werden.

Das verbleibende Reaktionsprodukt der ersten Stufe wird nun in eine Mischung aus Phosgen und vorzugsweise einem N,N-Dialkylcarbonsäureamid bzw. in die Suspension des Adduktes aus diesen Komponenten in verflüssigtem Phosgen vorzugsweise bei 40 bis 70°C so eingegeben, daß in der so entstehenden Reaktionsmischung der zweiten Reaktionsstufe stets das Phosgen bzw. ein Addukt aus Phosgen und dem N,N-Dialkylcarbonsäureamid im Überschuß vorhanden ist. Auch bei dieser Stufe wird der freiwerdende Chlorwasserstoff abgeleitet und gegebenenfalls isoliert. Nach Beendigung der Reaktion kann das überschüssige Phosgen in an sich bekannter Weise zurückgewonnen werden. Das verbleibende Reaktionsgemisch wird beispielsweise durch fraktionierte Destillation unter vermindertem Druck, vorzugsweise im Bereich von 0,1 bis 10 mbar, aufgearbeitet.

Im Vergleich zu den an sich bekannten einzelnen Reaktionsschritten erhält man nach dem erfindungsgemäßen Verfahren sehr reine Sulfonsäurechloride in hohen Ausbeuten, bezogen sowohl auf die eingesetzt aromatische Verbindung als auch auf die verwendete Chlorsulfonsäure.

Ein besonderer technischer und ökologischer Vorteil des erfindungsgemäßen Verfahrens liegt gegenüber dem bekannten Verfahren insbesondere auch darin, daß as möglich ist, das Reaktionsgemisch destillativ aufzuarbeiten.

Man kann bei Normaldruck, vorzugsweise bei vermindertem Druck bis zu etwa 10 Torr, das überschüssige Phosgen, gegebenenfalls das Lösungsmittel und gegebenenfalls die nicht umgesetzte Chlorsulfonsäure oder die nicht umgesetzte aromatische Verbindung abdestillieren und wiederverwenden. Anschließend kann man aus dem Rückstand das Sulfonsäurechlorid durch Kristallisation oder durch Destillation unter vermindertem Druck, vorzugsweise bei 0,1 bis 15 Torr, insbesondere bei 0,5 bis 10 Torr, in hoher Reinheit erhalten.

Die nach dem erfindungsgemäßen Verfahren mögliche destillative Aufarbeitung erübrigt eine Aufarbeitung mit Wasser und vermeidet den mit erheblichen Kostenaufwendungen verbundenen Zwangsanfall an Dünnsäure.

Beispiel 1 bis 6

116,5 g (1,0 Mol) Chlorsulfonsäure und 5,0 g (0,068 Mol) Dimethylformamid werden im Reaktionskolben vorgelegt und in diese Mischung werden 78, 1 g (1,0 Mol) Benzol innerhalb von vier Stunden eingetropft. Die Reaktionsmischung wird bis zum Ende der Gasentwicklung gerührt und anschließend wird nicht umgesetztes Benzol im Wasserstrahlvakuum abdestilliert.

15 g (0,2 Mol) Dimethylformamid werden in einem zweiten Kolben vorgelegt und dazu ca. 15 ml kondensiertes Phosgen gegeben. Dann wird die vom Benzol befreite Reaktionsmischung der ersten Stufe innerhalb von vier Stunden in den zweiten Reaktionskolben eingetropft. Gleichzeitig wird Phosgen so eindosiert, daß es—erkennbar am starken Rückfuß—immer im überschuß gegenüber Benzolsulfonsäure in der Reaktionsmischung der zweiten Stufe vorhanden ist. Nach Ende des Zutropfens wird die Mischung bis zum Ende der Gasentwicklung gerührt. Phosgenreste und andere flüchtige Bestandteile der Reaktionsmischung werden im Wasserstrahlvakuum abgezogen; der Rückstand wird dann im Ölpumpenvakuum über eine verspiegelte Kolonne (3 cm x 30 cm) fraktioniert destilliert.

Weitere Einzelheiten zur Durchführung und zu den Ergebnissen der Versuche sind der nachstehenden Tabelle zu entnehmen. Die Gesamtausbeuten sind auf den Sulfonierungsumsatz—berechnet nach der zurückgewonnenen Benzolmenge—bezogen.

Tabelle zu den Beispielen 1 bis 6

| Beispiel | Sulfonierungs-temperatur (°C) | Sulfonierungs-umsatz (%) | Phosgenierungs-temperatur (°C) | Produktfraktionen | Gesamt-ausbeute |
|---|---|---|---|---|---|
| 1 | 20 | 80,5 | 20 | I 121,1 g (98,7 %ig) | 88,6 % |
| | | | | II 14,8 g (43,5 %ig) | |
| 2 | 20 | 82,7 | 60 | I 122,8 g (98,6 %ig) | 86,8 % |
| | | | | II 11,6 g (49,1 %ig) | |
| 3 | 40 | 77,7 | 40 | I 116,2 g (99,4. %ig) | 88,2 % |
| | | | | II 8,7 g (62,9 %ig) | |
| 4 | 40 | 77,4 | 60 | 127,7 g (95,4 %ig) | 92,7 % |
| 5 | 60 | 76,7 | 60 | I 123,0 g (99,0 %ig) | 92,4 % |
| | | | | II 15,0 g (22,9 %ig) | |
| 6 | 80 | 83,7 | 60 | I 123,6 g (98,9 %ig) | 86,2 % |
| | | | | II 8,3 g (62,1 %ig) | |

Beispiel 7

Beispiel 7 wurde bei gleicher Ansatzgröße analog Beispiel 1 bis 6 durchgeführt. Als Sulfonierungshilfsmittel wurde Phosphorsäure (5 g) an Stelle von Dimethylformamid verwendet. Die erste Stufe (Sulfonierung) wurde bei 70°C, die zweite Stufe (Phosgenierung) bei 60°C durchgeführt. Nach Destillation erhielt man:

126,1 g Hauptlauf (99,5%ig) und
5,3 g Nachlauf (81.9%ig)

Die Gesamtausbeute beträgt 86,4% der Theorie, bezogen auf umgesetztes Benzol (Sulfonierungsumsatz: 85,1%).

Beispiel 8 bis 10

Die Beispiele 8 bis 10 wurden analog Beispiel 4 durchgeführt, jedoch wurde die Menge des als Sulfonierungszusatz verwendeten Dimethylformamid (DMF) variiert. Einzelheiten sind der folgenden Tabelle zu entnehmen:

Tabelle zu den Beispielen 8 bis 10

| Beispiel | Ansatz | Zusatz DMF | Sulfonierungsumsatz | Produktfraktionen | Gesamtausbeute |
|---|---|---|---|---|---|
| 8 | 1,0 Mol | 1,5 g (0,02 Mol) | 72,5 % | I 112 g (98,3 %ig) | 89,0 % |
| | | | | II 10 g (38,5 %ig) | |
| 9 | 1,0 Mol | 3.7 g (0,05 Mol) | 79,5 % | I 123,5 g (99,1 %ig) | 91,3 % |
| | | | | II 8,5 g (68,8 %ig) | |
| 10 | 1,0 Mol | 5,9 g (0,08 Mol) | 77,0 % | I 119,5 g (98,4 %ig) | 90,4 % |
| | | | | II 11 g (49,3 %ig) | |

Beispiel 11

Beispiel 11 wurde analog Beispiel 9 durchgeführt mit der Abweichung, daß Chlorsulfonsäure im Überschuß (122,5 g; 1,05 Mol) verwendet wurde. Nach Destillation erhielt man

128 g Hauptlauf (99,2%ig) und
5,5 g Wachlauf (71,8%ig).

Die Gesamtausbeute beträgt 90,8% der Theorie, bezogen auf umgesetztes Benzol (Sulfonierungsumsatz: 81,6%).

Beispiel 12

Beispiel 12 wurde analog Beispiel 1 bis 6 durchgeführt, jedoch wurde als Sulfonierungshilfsmittel Phosphorsäure (3 g) verwendet; die Sulfonierung wurde bei 40°C, die Phosgenierung bei 60°C durchgeführt.
Nach Destillation erhielt man

125 g Benzolsulfochlorid 99,3 %ig.

Die Gesamtuasbeute beläuft sich auf 91,4% der Theorie, bezogen auf umgesetztes Benzol (Sulfonierungsumsatz: 77,0 %).

**Patentansprüche**

1. Verfahren zur Herstellung von Sulfonsäurechloriden der Formel

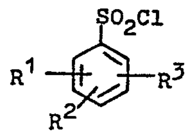 (I)

worin

$R^1$, $R^2$ und $R^3$ gleich oder verschieden sind und Wasserstoff, einen niederen Alkyl- oder einen Cycloalkylrest, Halogen, Aryl, Aralkyl, Arylether oder einen Rest $-SO_2Cl$, $-SO_2$-Aryl,

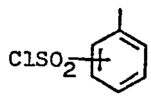 , 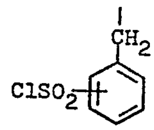 , 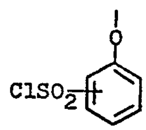 oder 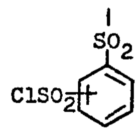

bedeuten

oder wo benachbarte Reste

$R^1$ und $R^2$ zu einem gegebenenfalls durch eine Sulfonsäurechloridgruppe substituierten cycloaliphatischen oder aromatischen carbocyclischen Ring verknüpft sind, durch Umsetzung von aromatischen Verbindungen dr Formel

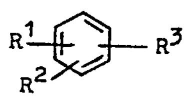 (II)

worin

$R^1$, $R^2$ und $R^3$ die oben angegebene Bedeutung haben, mit Chlorsulfonsäure und Phosgen dadurch gekennzeichnet, daß man zuerst die aromatische Verbindung mit Chlorsulfonsäure in einer molaren Menge von etwa 1:1 bis zu 1:1,2, gegebenenfalls in Gegenwart eines Lösungsmittels und gegebenenfalls in Gegenwart einer Verbindung, die gegenüber Chlorsulfonsäure als Lewis-Base wirksam werden kann, zur Reaktion bringt und dann in Gegenwart eines an sich bekannten Katalysators mit Phosgen umsetzt.

2. Verfahren nach Anspruch 1, dadurch gekennzeichnet, daß die aromatische Verbindung und Chlorsulfonsäure im molaren Verhältnis 1:1 eingesetzt werden.

3. Verfahren nach Anspruch 1 und 2, dadurch gekennzeichnet, daß Chlorsulfonsäure im Überschuß bis zu 20 Mol-%, bezogen auf die aromatische Verbindung, eingesetzt wird.

4. Verfahren nach Anspruch 1 bis 3, dadurch gekennzeichnet, daß das in der ersten Verfahrensstufe aus der aromatischen Verbindung und Chlorsulfonsäure erhaltene Reaktionsgemisch in der zweiten Verfahrensstufe so mit Phosgen umgesetzt wird, daß im Reaktionsgemisch der zweiten Verfahrensstufe das Phosgen im Überschuß gegenüber dem Reaktionsprodukt der ersten Verfahrensstufe vorhanden ist.

## Claims

1. Process for the preparation of sulphonic acid chlorides of the formula

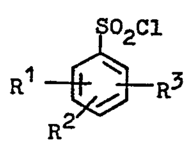 (I)

wherein

$R^1$, $R^2$ and $R^3$ are identical or different and denote hydrogen, a lower alkyl radical or a cycloalkyl radical, halogen, aryl, aralkyl, aryl ether or a radical $-SO_2Cl$, $-SO_2$-aryl,

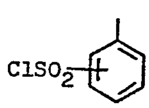 , 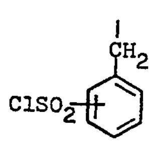 , 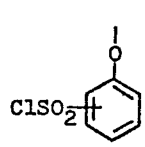 or 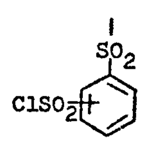

or wherein

Adjacent radicals $R^1$ and $R^2$ are linked to form a cycloaliphatic or aromatic carbocyclic ring which

is optionally susbstituted by a sulphonic acid chloride group, by reacting aromatic compounds of the formula

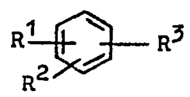

(II)

wherein

$R^1$, $R^2$ and $R^3$ have the meaning indicated above, with chlorosulphonic acid and phosgene, characterized in that the aromatic compound is first reacted with chlorosulphonic acid in a molar amount of approximately 1:1 to 1:1.2, optionally in the presence of a solvent and optionally in the presence of a compound which can act as a Lewis base towards chlorosulphonic acid and the product is then reacted with phosgene in the presence of a known catalyst.

2. Process according to Claim 1, characterised in that the aromatic compound and chlorosulphonic acid are employed in a molar ratio of 1:1.

3. Process according to Claims 1 and 2, characterised in that chlorosulphonic acid is employed in an excess of up to 20 mol%, relative to the aromatic compound.

4. Process according to Claims 1 and 3, characterised in that the reaction mixture obtained from the aromatic compound and chlorosulphonic acid in the first process stage is reacted with phosgene in the second process stage in a manner such that phosgene is present in the reaction mixture of the second process stage in excess, relative to the reaction product from the first process stage.

**Revendications**

1. Procédé de fabrication de chlorures d'acides sulfoniques de formule:

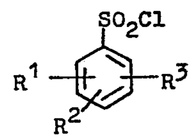

(I)

dans laquelle

$R^1$, $R^2$ et $R^3$ sont identiques ou différents et représentent de l'hydrogène, un radical alcoyle inférieur ou un radical cycloalcoyle, halogéno, aryle, aralcoyle, aryléther ou un radical $—SO_2Cl$, $—SO_2$-aryle,

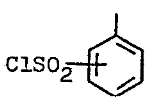 , 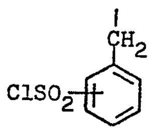 , 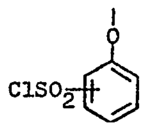 ou 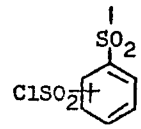

ou bien des radicaux voisins

$R^1$ et $R^2$ sont reliés à un noyau carbocyclique cycloaliphatique ou aromatique éventuellement substitué par un groupe chlorure d'acide sulfonique,

par réaction de composés aromatiques de formule:

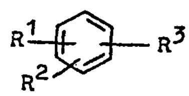

(II)

dans laquelle

$R^1$, $R^2$ et $R^3$ ont la signification indiquée plus haut, avec de l'acide chlorosulfonique et du phosgène, caractérisé en ce que tout d'abord on fait réagir le composé aromatique avec l'acide chlorosulfonique en une quantité molaire d'environ 1:1 à 1:1,2, éventuellement en présence d'un solvant et éventuellement en présence d'un composé que peut se comporter par rapport à l'acide chlorosulfonique comme une base de Lewis, puis on faite réagir avec du phosgène en présence d'un catalyseur connu en lui-même.

2. Procédé selon la revendication 1, caractérisé en ce qu'on utilise le composé aromatique et l'acide chlorosulfonique dans le rapport molaire de 1:1.

3. Procédé selon l'une quelconque des revendications 1 et 2, caractérisé en ce qu'on utilise l'acide chlorosulfonique en un excès atteignant jusqu'à 20 moles % par rapport au composé aromatique.

4. Procédé selon l'une quelconque des revendications 1 à 3, caractérisé en ce que le mélange de réaction obtenu dans le premier stade opératoire à partir du composé aromatique et de l'acide chlorosulfonique est mis à réagir dans le second stade opératoire avec du phosgène en sorte que, dans le mélenge de réaction du second stade opératoire, le phosgène soit présent en excès par rapport au produit de réaction du premier stade opératoire.